# EUROPÄISCHE PATENTSCHRIFT

(11) **EP 0 384 155 B1**
(45) Veröffentlichungstag und Bekanntmachung des Hinweises auf die Patenterteilung: **13.07.1994**
(21) Anmeldenummer: 90101574.3
(22) Anmeldetag: 26.01.1990
(51) Int. Cl.: A61M 5/172

(54) **Kombination aus einem Medizintechnische Gerät mit redundant aufgebauter Steuereinheit zur Infusionstherapie oder Blutbehandlung und einem Programmiergerät**
Combination of a medical device with a redundant controller for infusion therapy or blood treatmentand a programming device
Combination d'un dispositif médical avec contrôleur superflu pour perfusion thérapeutique ou traitement de sang et d'un dispositif de programmation

(30) Priorität: 22.02.1989 DE 3905350
(43) Veröffentlichungstag der Anmeldung: 29.08.1990
(73) Patentinhaber: B. Braun Melsungen AG, 34209 Melsungen (DE)
(72) Erfinder: Steger, Jürgen, Dipl.-Ing, D-3582 Felsberg (DE); Seidel, Franz, Dipl.-Ing., D-3500 Kassel (DE); Heitmeier, Rolf, Dipl.-Ing., D-3507 Baunatal (DE)
(74) Vertreter: Selting, Günther, Dipl.-Ing.

(56) Entgegenhaltungen:
- EP-A- 0 319 272
- GB-A- 2 056 133
- US-A- 4 624 661
- MEDIZINTECHNIK, Band 105, April 1985, Seiten 118-124; R. HEITMEIER et al.: "Sicherheitstechnik bei einem mikroprozessorgesteuerten Hämodialysegerät"

## Beschreibung

Die Erfindung betrifft eine Kombination aus einem medizintechnischen Gerät zur Infusionstherapie oder Blutbehandlung mit einer redundant aufgebauten Steuereinheit und eines an die Steuereinheit auschließbaren Programmiergeräts.

Medizintechnische Geräte, wie beispielsweise Dialysegeräte und Infusionspumpen, müssen sehr hohen sicherheitstechnischen Anforderungen genügen, wobei insbesondere sichergestellt werden muß, daß in der Steuereinheit nicht durch Ausfall von Komponenten oder durch fehlerhafte Datenübertragung eine Gefährdung des Patienten auftreten kann. Aus medizintechnik 1985, 118-124, ist es bekannt, bei einem mikroprozessorgesteuerten Hämodialysegerät die Steuereinheit redundant auszubilden, wobei ein Mikroprozessor als Funktionsprozessor die Regelung und Überwachung des Dialysegeräts durchführt, während ein zweiter Mikroprozessor als Kontrollprozessor vorgesehen ist, der die gleichen Daten empfängt wie der Funktionsprozessor und der den Funktionsprozessor kontrolliert. Es ist auch möglich, Funktionsprozessor und Kontrollprozessor als diversitäre Systeme auszubilden, d.h. für diese Mikroprozessoren unterschiedliche Prozessortypen zu verwenden, die eventuell noch mit unterschiedlicher Software programmiert sind, so daß eventuell unerkannt gebliebene systembedingte Fehler, die nur in einem der unterschiedlich aufgebauten Prozessorsysteme vorkommen, erkannt werden. Das mit den redundant aufgebauten Steuereinheiten erzielbare Sicherheitsniveau ist hoch. Allerdings haben die Steuereinheiten derartiger medizintechnischer Geräte eine Eingabeeinheit, die nur ganz wenige Eingabeelemente, z.B. Tasten, aufweist, um Fehlbedienungen auszuschließen. Die Programmparameter sind zum größten Teil in der Steuereinheit gespeichert. Sie sind vom Hersteller eingegeben und können vom Betreiber normalerweise nicht verändert werden. Die Veränderung von sicherheitsrelevanten Daten ist mit erheblichen Risiken verbunden. Falsch eingegebene oder durch verarbeitete oder durch Störungen veränderte Daten können gravierende Auswirkungen auf den Betrieb des medizintechnischen Gerätes haben und eine erhebliche Gefährdung für den Patienten darstellen, der mit diesem Gerät behandelt wird. Eine redundant aufgebaute Steuereinheit wird auch als zweikanaliges System bezeichnet. Wenn die Steuereinheit zweikanalig ausgebildet ist, müßte zur Aufrechterhaltung des Sicherheitsstandards auch dasjenige Gerät, mit dem sicherheitsrelevante Daten in die Steuereinheit eingegeben oder verändert werden, ebenfalls zweikanalig ausgebildet sein. Ein solches zweikanaliges Programmiergerät wäre aufwendig und seine hohen Anschaffungskosten würden den Einsatz solcher Programmiergeräte nur dann rechtfertigen, wenn mit einem Programmiergerät zahlreiche medizintechnische Geräte bearbeitet werden können. Für einzelne medizintechnische Geräte würde sich ein derart aufwendiges Programmiergerät nicht lohnen.

Aus GB-A-2 056 133 ist eine Vorrichtung zur Überwachung und/oder Kontrolle der Operationen eines Computers von einer entfernten Stelle aus bekannt. Bei dieser Vorrichtung wird ein Benutzerplatz mit dem Arbeitsplatz eines Beratungszentrums über eine Telefonleitung als Datenleitung verbunden. Der Benutzer verfügt über eine CPU und ein Display-Terminal und der Arbeitsplatz im Beratungszentrum verfügt ebenfalls über ein Display-Terminal, an dem die selben Daten angezeigt werden können, wie an dem Display-Terminal des Benutzerplatzes. Auf diese Weise kann der Berater die Eingaben des Benutzers und die Reaktionen von dessen CPU überprüfen und beratend eingreifen.

Der Erfindung liegt die Aufgabe zugrunde, eine Kombination eines medizintechnischen Geräts und eines Programmiergeräts zu schaffen, die ohne Verringerung des Sicherheitsstandards auf einfache Weise eine Programmierung des medizintechnischen Geräts ermöglicht, ohne daß an das Sicherheitsniveau des Programmiergeräts besondere Anforderungen gestellt werden müßten.

Die Lösung dieser Aufgabe erfolgt erfindungsgemäß mit den Merkmalen des Patentanspruchs 1.

Bei der erfindungsgemäßen Kombination ist ein Programmiergerät vorgesehen, das für einen Datenaustausch mit der Steuereinheit geeignet ist, so daß von dem Programmiergerät Daten in die Steuereinheit übertragen werden können, wenn die Steuereinheit nicht gerade einen Steuerprozeß für einen an das Gerät angeschlossenen Patienten durchführt. Das Programmiergerät kann eine umfangreiche Eingabeeinheit in Form einer Tastatur aufweisen und auch eine vielseitige Anzeigeeinrichtung in Form eines Bildschirms. Während Eingabeeinheit und Anzeigeeinrichtung der Steuereinheit relativ einfach ausgebildet sind und nur wenige Funktionen bzw. eine relativ geringe Anzeigekapazität haben, kann das Programmiergerät mit einer vielseitigen Eingabeeinheit und Anzeigeeinrichtung ausgestattet sein. Als Programmiergerät eignet sich beispielsweise ein üblicher Personal Computer. Die in die Steuereinheit zu übertragenden Daten können in einem Speicher des Programmiergeräts vorgespeichert sein, sie können aber auch von dem Programmiergerät aufgrund von Programmen errechnet und zusammengestellt werden. Schließlich ist es auch möglich, diese Parameter über die Eingabeeinheit manuell in das Programmiergerät einzugeben.

Die Besonderheit der Erfindung besteht darin, daß nach erfolgter Übertragung der Daten vom Programmiergerät zur Steuereinheit ein Prozeß durchgeführt werden kann, bei dem die übertragenen Daten, die auch noch im Programmiergerät gespeichert sind, sowohl an der Anzeigeeinrichtung des Programmiergeräts als auch an derjenigen der Steuereinheit gleichzeitig angezeigt werden. In einem Vergleich kann der Anwender kontrollieren, ob die in das Programmiergerät eingegebenen oder im Programmiergerät aufbereiteten Daten korrekt in die zweikanalige Steuereinheit übertragen worden sind und in den dort zusätzlich vorgesehenen Applikationsspeichern auch korrekt abgespeichert worden sind. Etwaige Fehler, die im Datenübertragungskanal zwischen Programmiergerät und Steuereinheit auftreten, werden durch den Datenvergleich der Anzeigeeinrichtungen auf einfache Weise erkannt. Auch solche Fehler, die bei der Einspeicherung innerhalb der Steuereinheit entstehen, werden erkannt.

Die Erfindung ermöglicht die Anwendung eines üblichen Personal Computers mit Bildschirm und Tastatur als Programmiergerät, ohne diese Funktionselemente in jede Steuereinheit implementieren zu müssen. Dies bedeutet eine erhebliche Kosten- und Gewichtseinsparung. Der Benutzer kann im Programmiergerät verschiedene Verfahrensabläufe zusammenstellen und modifizieren und die Verfahrensabläufe vor ihrer Übertragung in die Steuereinheit auch am Bildschirm sichtbar machen, kontrollieren und ggf. verändern. Dabei kann der Benutzer einige der Parameter eingeben, während andere Parameter von dem Programmiergerät aus den eingegebenen Parametern errechnet werden. Wenn der gesamte benötigte Parametersatz im Programmiergerät vollständig vorliegt, kann z.B. in einer Betriebspause des medizintechnischen Geräts die Datenübertragung in die Steuereinheit erfolgen. Bevor die Steuereinheit, in die die Daten übertragen wurden, mit den neuen Daten in Betrieb genommen wird, muß durch den Benutzer Punkt für Punkt ein Vergleich der an den beiden Anzeigeeinrichtungen angezeigten Daten erfolgen, d.h. der Benutzer überzeugt sich davon, daß die Parameter, die im Programmiergerät enthalten sind, korrekt in der Steuereinheit abgespeichert worden sind. Erst wenn diese Vergleichsprüfung abgeschlossen ist, wird das medizintechnische Gerät am Patienten in Betrieb genommen.

Bei Betrieb des medizintechnischen Geräts am Patienten kann das Programiergerät an die Steuereinheit angeschlossen bleiben. Zweckmäßigerweise ist jedoch vorgesehen, daß beim Betrieb am Patienten die Datenübertragung vom Programmiergerät zur Steuereinheit unterbrochen ist, so daß das Programmiergerät in gar keiner Weise auf den laufenden Steuer- und Regelprozeß einwirken kann. Das Programmiergerät kann allerdings beobachtend an der Funktion der Steuereinheit teilnehmen, d.h. es ist ein Datenfluß von der Steuereinheit zum Programmiergerät möglich. Auf diese Weise kann der Benutzer über den Bildschirm des Programmiergeräts eventuell zusätzliche Informationen über den ablaufenden Prozeß erhalten, die an der sehr einfach aufgebauten Anzeigeeinrichtung der Steuereinheit nicht angezeigt werden können.

Die Applikationsspeicher, die die vom Programmiergerät an die Steuereinheit übertragenden Daten (Anweisungsliste, Parameter, Konstanten, Programme) aufnehmen, sind löschbare Speicher, da es möglich sein muß, ihren Inhalt zu löschen und durch einen neuen Inhalt zu ersetzen. Andererseits muß sichergestellt sein, daß bei einem Ausfall der Stromversorgung der Steuereinheit der Inhalt dieser Speicher nicht gelöscht wird. Die Applikationsspeicher sind daher z.B. batteriegepufferte Schreib-Lese-Speicher.

Um dem Benutzer die Richtigkeitskontrolle der übertragenen Daten zu erleichtern, ist es zweckmäßig, die Steuerung derart vorzunehmen, daß auf einen Tastendruck am Programmiergerät hin die Fortschaltung der Anzeige einander entsprechender Daten an beiden Anzeigeeinrichtungen gleichzeitig erfolgt. Auf diese Weise ist sichergestellt, daß an beiden Anzeigeeinrichtungen stets die zu vergleichenden Daten angezeigt werden, so daß die Gefahr, daß der Benutzer nicht zusammengehörende Daten miteinander vergleicht, ausgeschlossen ist. Der Anwender muß die an den Anzeigeeinrichtungen angezeigten Daten auf Übereinstimmung prüfen und dies durch Tastendruck quittieren. Dadurch werden Fehler, die im System, der Übertragungsstrecke oder dem Programmiergerät entstehen könnten, erkannt.

Eine weitere Erleichterung in bezug auf die Richtigkeitskontrolle der übertragenen Daten stellt der Vergleich einer Prüfsumme mit einem Erwartungswert dar. Hierzu wird in dem Programmiergarät und in der Steuereinheit jeweils eine Prüfsumme gebildet und am Programmiergerät und an der Steuereinheit angezeigt. Der Anwender vergleicht die beiden Prüfsummen und quittiert die Übereinstimmung der Prüfsummen am Steuergerät. Bei dieser Vorgehensweise reduziert sich der Vergleichs- und Quittiervorgang auf einen einmaligen Vorgang.

Die Erfindung ist bei medizinischen Geräten zur Infusionstherapie oder Blutbehandlung anwendbar, die mit einer mindestens zweikanaligen Steuereinheit ausgestattet sind. Sie eignet sich insbesondere für solche medizinischen Geräte, die neben einem Programmablauf auch Überwachungsfunktionen durchführen. Die Daten können Konstanten (Grenzwerte) sein oder auch Parameter (Förderrate) oder auch eine Anweisungsliste. Diese kann Zeitprofile oder Algorithmen, d.h. Verknüpfungsfunktionen von Ereignissen beinhalten. Diese Anweisungsliste besteht vorzugsweise aus Sprachelementen einer Hochsprache. Diese Sprachelemente eignen sich in besonderer Weise für den Anwendervergleich und können von den in der Steuereinheit implementierten Interpretern in die vorgegebene Applikation umgesetzt werden. Durch den Anschluß des Programmiergeräts werden die Anwendungsmöglichkeiten des medizintechnischen Gerätes ohne zusätzliche Eingabeelemente an der Steuereinheit wesentlich erweitert. Die Daten können auch Programme sein, welche nach der Übertragung in den Applikationsspeicher den Befehlsvorrat der Interpreter ergänzen und als Teile der Interpreter arbeiten. Eine Modifikation der Interpreter ist somit ohne Wechsel der Festwertspeicher möglich (Bootstrapfunktion).

Im folgenden wird unter Bezugnahme auf die Zeichnungen ein Ausführungsbeispiel der Erfindung näher erläutert.

Es zeigen:

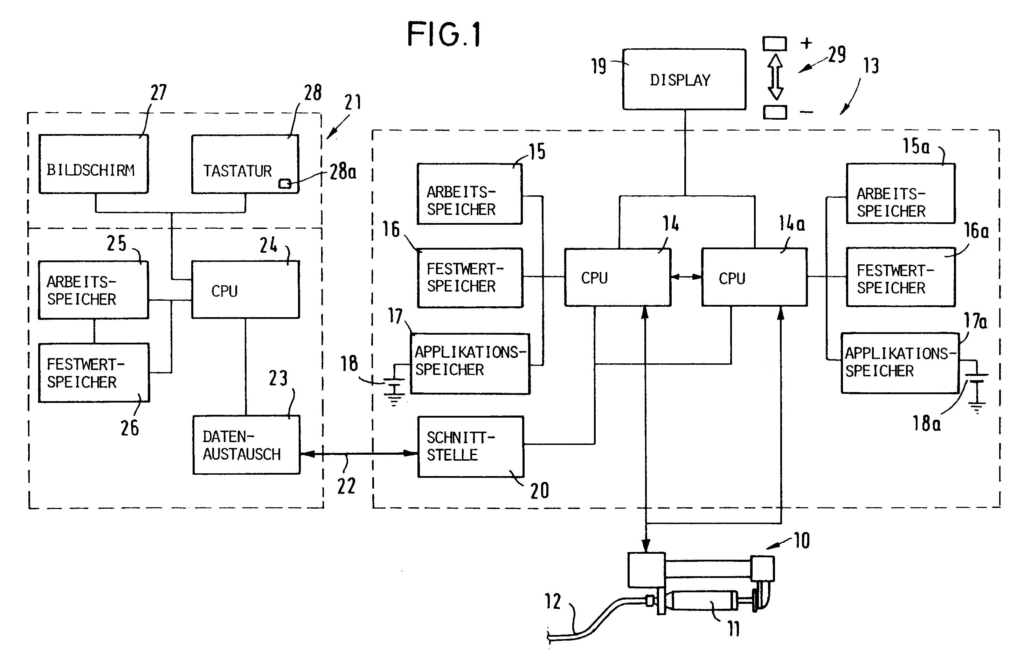

- Fig. 1: eine schematische Darstellung der Steuereinheit und des Programmiergerätes,
- Fig. 2: eine Tabelle von Parametersätzen, die vom Programmiergerät an die Steuereinheit übertragen werden und
- Fig. 3: ein zeitliches Profil eines Steuervorgangs, der im Programmiergerät vorbereitet werden kann und dessen Daten an die Steuereinheit übertragen werden können.

In den Zeichnungen ist als Anwendungsbeispiel für das medizinische Gerät eine Infusionspumpe 10 dargestellt, die den Inhalt einer Spritze 11 ausdrückt, um durch einen Schlauch 12 eine Flüssigkeit einem Patienten zuzuführen. Die Infusionspumpe 10 weist einen (nicht dargestellten) Antriebsmotor auf, der durch die Steuereinheit 13 gesteuert ist. Außerdem sind an der Infusionspumpe 10 Sensoren vorhanden, deren Signale an die Steuereinheit 13 übertragen werden.

Die Steuereinheit 13 ist als redundantes zweikanaliges System aufgebaut. Sie enthält sämtliche Komponenten zweifach, wobei die Elemente eines Paares nicht notwendigerweise gleich aufgebaut sind, aber die gleichen Funktionen ausführen. Jeder der beiden Kanäle enthält eine zentrale Prozessoreinheit (CPU) 14 bzw. 14a, die mit einem Arbeitsspeicher 15 bzw. 15a, einem Festwertspeicher 16 bzw. 16a und einem Applikationsspeicher 17 bzw. 17a zusammenarbeitet. Die beiden Prozessoreinheiten 14 und 14a sind untereinander verbunden, um ihre Daten und Arbeitsabläufe auf Koinzidenz zu überprüfen. Wird keine Koinzidenz festgestellt, dann wird Alarm erzeugt.

Jeder der beiden Kanäle enthält einen Applikationsspeicher 17 bzw. 17a, der durch eine Batterie 18 bzw. 18a gepuffert ist. Dieser Applikationsspeicher ist ein Schreib-Lese-Speicher. Die in ihm enthaltenen Daten werden bei Stromausfall durch die Batterie 18 bzw. 18a erhalten.

Die Steuereinheit 13 weist eine (einzige) Anzeigeeinrichtung 19 auf, bei der es sich um ein alphanumerisches Display handelt, an dem ein Datensatz aus z.B. 16 Ziffern oder Buchstaben angezeigt werden kann.

Ferner ist an der Steuereinheit 13 eine Stelleinrichtung 29 vorgesehen, durch die es möglich ist, einen bestimmten Parameter, z.B. die Infusionsrate, manuell zu verändern. Diese Stelleinrichtung 29 besteht aus einer Plustaste und einer Minustaste, durch deren Drücken die an der Anzeigeeinrichtung 19 angezeigte Infusionsrate nach oben oder unten verändert werden kann. Die Stelleinrichtung 29 ist auf ein Mindestmaß von Verstellmöglichkeiten beschränkt, damit der Infusionsbetrieb an der Steuereinheit ohne zusätzliches Programmiergerät verändert werden kann, andererseits aber Bedienungsfehler, die bei einem komplexen Eingabesystem leicht möglich wären, vermieden werden.

Der Steuereinheit ist eine Schnittstelle 20 für das Zusammenarbeiten mit dem Programmiergerät 21 vorgesehen. Das Programmiergerät 21, bei dem es sich um einen Personal Computer handelt, weist eine entsprechende Schnittstelle 23 auf. Durch einen Übertragungskanal 22, z.B. durch ein Datenübertragungskabel, können die Schnittstellen 23 und 20 untereinander verbunden werden.

Das Programmiergerät 21 weist in üblicher Weise eine zentrale Prozessoreinheit (CPU) 24, einen Arbeitsspeicher 25 und einen Festwertspeicher 26 auf, sowie ferner einen Bildschirm 27 und eine manuelle Eingabeeinheit 28 in Form einer Tastatur. An der Tastatur 28 ist eine spezielle Taste 28a vorgesehen, deren Funktion noch erläutert wird.

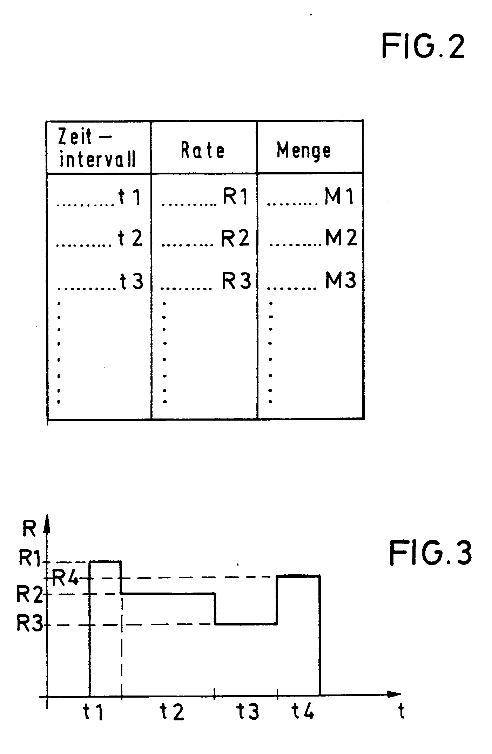

Mit Hilfe der Eingabeeinheit 28 können in das Programmiergerät Daten für den Betrieb der Infusionspumpe eingegeben werden. Gemäß Fig. 2 besteht ein Datensatz aus den Angaben über das Zeitintervall tn, die Infusionsrate Rn und die Infusionsmenge Mn, wobei n der Index des jeweiligen Datensatzes ist. Vom Benutzer brauchen nur jeweils zwei der drei Daten eingegeben zu werden, während der dritte Wert von dem Programmiergerät errechnet werden kann.

Fig. 3 zeigt ein mögliches Infusionsprofil, bei dem über die Zeitintervalle t1 bis t4 unterschiedliche Infusionsraten R1 bis R4 realisiert werden. Die Infusionsrate R gibt die dem Patienten zugeführte Flüssigkeitsmenge pro Zeiteinheit an und die Infusionsmenge M ist die dem Patienten insgesamt zugeführte Flüssigkeitsmenge. Das in Fig. 3 dargestellte Infusionsprofil kann an dem Bildschirm 27 graphisch angezeigt werden, so daß der Benutzer dieses Infusionsprofil optisch erkennen und durch Umprogrammierung verändern kann, bevor die entsprechenden Daten (Parameter) in die Steuereinheit 13 übertragen werden. Die in Fig. 2 dargestellte Tabelle von Parametern wird in dem Arbeitsspeicher 25 des Programmiergeräts 21 gespeichert.

Die Steuereinheit 13 enthält einen Sensor, der feststellt, ob die Antriebseinheit 10 in Funktion ist. Eine Datenübertragung von dem Programmiergerät 21 zur Steuereinheit 13 ist nur dann möglich, wenn die Antriebseinheit 10 nicht in Betrieb ist. Eine solche Datenübertragung wird vom Benutzer durch einen Tastendruck an der Tastatur 28 ausgelöst. Die im Arbeitsspeicher 25 gespeicherten Daten werden dann in die Applikationsspeicher 17 und 17a übertragen. Nach Beendigung dieser Datenübertragung muß zunächst ein Vergleich durchgeführt werden, um zu ermitteln, ob die Daten korrekt übertragen und gespeichert worden sind. Dies geschieht dadurch, daß die Taste 28a an der Tastatur des Programmiergeräts 21 gedrückt wird. Nach erstmaligem Druck der Taste 28a erscheint der erste in der Tabelle gemäß Fig. 2 enthaltenen Parameter an der Anzeigeeinrichtung 27 des Programmiergeräts 21. Der entsprechende Befehl wird auch zur Steuereinheit 13 übertragen, so daß der entsprechende Parameter, der in den Applikationsspeichern 17 und 17a enthalten ist, an der Anzeigeeinrichtung 19 angezeigt wird. Der Benutzer kann nun feststellen, ob die Anzeigen an den Anzeigeeinrichtungen 27 und 19 einander gleich sind. Wenn Übereinstimmung der Anzeigen festgestellt wurde, wird die Taste 28a noch einmal gedrückt, so daß zum nächsten Parameter fortgeschaltet wird. Auf diese Weise erfolgt durch Drücken der Taste 28a eine Fortschaltung der Parameter sowohl an der Anzeigeeinrichtung 27 als auch an der Anzeigeeinrichtung 19. Das Drücken der Taste 28a gilt als Quittierung der Tatsache, daß Übereinstimmung beider Anzeigen festgestellt wurde. Erst wenn alle Daten auf diese Weise angezeigt und quittiert worden sind, ist der Vergleich abgeschlossen.

Das Programmiergerät 21 kann auch nach Beendigung der Datenübertragung an die Steuereinheit 13 angeschlossen bleiben. Wenn die Antriebseinheit 10 in Betrieb ist, wird dies durch die zentralen Prozessoreinheiten 14 und 14a festgestellt und diese bewirken, daß eine Datenübertragung nur von der Steuereinheit 13 zum Programmiergerät 21 möglich ist. Das Programmiergerät 21 hat in diesem Zustand nur eine Beobachterfunktion.

Die Parameter t, R und M sind hier aus Gründen der einfacheren Erläuterung nur als Beispiele angegeben. Zusätzlich können weitere Daten eingegeben werden, beispielsweise die maximale Infusionsmenge pro Zeiteinheit, Infusionsratenveränderung, Anweisungslisten u.dgl.

## Patentansprüche

1. Kombination aus
einem medizinischen Gerät zur Infusionstherapie oder Blutbehandlung, mit einer redundant aufgebauten Steuereinheit (13), die Festwertspeicher (16,16a), die Interpreter enthalten, welche aus eingegebenen Daten die vorgegebene Applikation bilden, und Applikationsspeicher (17,17a) aufweist und deren Komponenten in mindestens zweifacher Ausführung vorhanden und simultan betrieben sind, und mit einer Anzeigeeinrichtung (19) zum Anzeigen von Daten aus der Steuereinheit (13)
und
einem Programmiergerät (21), das an die Steuereinheit (13) anschließbar ist und über eine manuelle Eingabeeinheit (28) und eine Anzeigeeinrichtung (27) verfügt,
wobei
Mittel zum Übertragen von Daten (Anweisungsliste, Parameter, Konstanten, Programme) aus dem Programmiergerät (21) in die Applikationsspeicher (17,17a) der Steuereinheit (13) und
Mittel zum gleichzeitigen Anzeigen der übertragenen Daten, einzeln oder in Gruppen, an den Anzeigevorrichtungen (19,27) von Steuereinheit (13) und Programmiergerät (21)
vorgesehen sind.

2. Kombination nach Anspruch 1, gekennzeichnet durch Mittel, die in der Steuereinheit (13) aus den übertragenen Daten eine Prüfsumme bilden und diese Prüfsumme die Anzeigeeinrichtung (19) der Steuereinheit (13) liefern.

3. Kombination nach Anspruch 1 oder 2, dadurch gekennzeichnet, daß die Applikationsspeicher (17,17a) der Steuereinheit (13) löschbare Schreib-Lese-Speicher sind.

4. Kombination nach einem der Ansprüche 1 bis 3, dadurch gekennzeichnet, daß die Steuereinheit (13) einen Sensor aufweist, der den Empfang von Daten des Programmiergeräts (21) durch die Steuereinheit (13) nur in den dafür vorgesehenen Betriebsphasen zuläßt.

5. Kombination nach einem der Ansprüche 1 bis 4, dadurch gekennzeichnet, daß die Steuereinheit (13) bei angeschlossenem Programmiergerät (21) derart betrieben ist, daß an ihrer Anzeigeeinrichtung (19) eine Fortschaltung angezeigter Daten synchron mit der Fortschaltung derselben Daten an der Anzeigeeinrichtung (27) des Programmiergeräts (21) erfolgt, wobei das Fortschalten am Programmiergerät (21) erfolgt.

6. Kombination nach einem der Ansprüche 1 bis 5, dadurch gekennzeichnet, daß die Steuereinheit (13) derart ausgebildet ist, daß sie nach Eingabe neuer Daten durch das Programmiergerät (21) erst dann in Betrieb gesetzt werden kann, wenn alle übertragenen Daten, bzw. die entsprechenden Prüfsummen, nacheinander an den Anzeigeeinrichtungen (19,27) angezeigt worden sind und für jede Anzeige eine Quittierung erfolgt ist.

## Claims

1. Combination of a medical apparatus for infusion therapy or blood treatment, with a redundantly constructed control unit (13) comprising read-only memories (16,16a) including interpreters for forming the predetermined application amounts from input data, and application amount memories (17,17a) and whose components are provided at least in duplicate and operated simultaneously, and with a display means (19) for displaying data from the control unit (13)
and
a programmer device (21) connectable to the control unit (13) and comprising a manual input unit (28) and a display unit (27),
wherein
means for transferring data (instruction list, parameters, constants, programmes) from the programmer device (21) into the application memories (17,17a) of the control unit (13) and
means for simultaneously displaying the transferred data, individually or in groups, on the display devices (19,27) of the control unit (13) and the programmer device (21)
are provided.

2. The combination according to claim 1, characterized by means forming a check sum of the transferred data in the control unit (13) and supplying this check sum to the display means (19) of the control unit (13).

3. The combination according to claim 1 or 2, characterized in that the application amount memories (17,17a) of the control unit (13) are erasable read-write memories.

4. The combination of one of claims 1 to 3, characterized in that the control unit (13) comprises a sensor permitting the reception of data of the programmer device (21) through the control unit (13) only in the operation phases provided for this.

5. The combination of one of claims 1 to 4, characterized in that the control unit (13), when the programmer device (21) is connected thereto, is operated in such a manner that a propagation of displayed data is effected on its display means (19) simultaneously with the propagation of the same data on the display means (27) of the programmer device (21), the propagation being effected at the programmer device (21).

6. The combination of one of claims 1 to 5, characterized in that the control unit (13) is configured such that, after the input of the new data by the programmer device (21), it can only be activated when all transferred data or the corresponding check sums, respectively, have been displayed successively on the display means (19,27) and an acknowledgement for each display has been effected.

## Revendications

1. Combinaison
d'un appareil médical pour une thérapeutique par perfusion ou le traitement du sang, comportant une unité de commande (13) redondante, qui comprend des mémoires mortes (16, 16a) renfermant des interpréteurs qui, à partir des données introduites, forment l'application prédéfinie, ainsi que des mémoires d'application (17, 17a), et dont les composants sont présents au moins sous forme double et fonctionnent simultanément, l'appareil comportant un dispositif d'affichage (19) pour afficher des données de l'unité de commande (13),
et
d'un appareil de programmation (21) qui peut être raccordé à l'unité de commande (13) et dispose d'une unité de saisie manuelle (28) et d'un dispositif d'affichage (27),
des moyens étant prévus pour la transmission de données (liste d'instructions, paramètres, constantes, programmes) de l'appareil de programmation (21) vers les mémoires d'application (17, 17a) de l'unité de commande (13),
et des moyens étant prévus pour l'affichage simultané des données transmises, individuellement ou par groupes, sur les dispositifs d'affichage (19, 27) de l'unité de commande (13) et de l'appareil de programmation (21).

2. Combinaison selon la revendication 1, caractérisée par des moyens qui dans l'unité de commande (13), forment une somme de contrôle des données transmises, et délivrent cette somme de contrôle au dispositif d'affichage (19) de l'unité de commande (13).

3. Combinaison selon la revendication 1 ou 2, caractérisée en ce que les mémoires d'application (17, 17a) de l'unité de commande (13) sont des mémoires à lecture-écriture, effaçables.

4. Combinaison selon l'une des revendications 1 à 3, caractérisée en ce que l'unité de commande (13) comporte un capteur qui n'autorise la réception de données de l'appareil de programmation (21) par l'unité de commande (13), uniquement au cours des phases de fonctionnement prévues à cet effet.

5. Combinaison selon l'une des revendications 1 à 4, caractérisée en ce que l'unité de commande (13), lorsque l'appareil de programmation (21) y est raccordé, fonctionnent dans un mode tel, que sur son dispositif d'affichage (19) s'effectue une succession de données affichées, synchronisée avec la succession des mêmes données sur le dispositif d'affichage (27) de l'appareil de programmation (21), le déroulement de la succession s'effectuant sur l'appareil de programmation (21).

6. Combinaison selon l'une des revendications 1 à 5, caractérisée en ce que l'unité de commande (13) est agencée de manière telle, qu'après l'introduction de nouvelles données par l'appareil de programmation (21), elle ne peut être mise en service que lorsque toutes les données transmises, et les sommes de contrôle correspondantes, ont été affichées successivement sur les dispositifs d'affichage (19, 27), et qu'une confirmation a été effectuée pour chaque affichage.
